# EUROPÄISCHE PATENTSCHRIFT

(11) **EP 2 656 944 B1**
(45) Veröffentlichungstag und Bekanntmachung des Hinweises auf die Patenterteilung: **21.03.2018**
(21) Anmeldenummer: 13163034.5
(22) Anmeldetag: 10.04.2013
(51) Int. Cl.: B21K 23/00, A61L 31/02, A61L 31/14, A61F 2/91, B21C 37/15, B21C 23/08

(54) **Verfahren zur Herstellung eines Implantats**
Method for producing an implant
Procédé de fabrication d'un implant

(30) Priorität: 23.04.2012 US 201261636742 P
(43) Veröffentlichungstag der Anmeldung: 30.10.2013
(73) Patentinhaber: Biotronik AG, 8180 Bülach (CH)
(72) Erfinder: Bayer, Ullrich, 18211 Admannshagen-Bargeshagen (DE); Block, Bernd, 18055 Rostock (DE); Lootz, Daniel, 18119 Rostock (DE); Hannemann, Jan, 18057 Rostock (DE); Venohr, Nils, 18057 Rostock (DE)
(74) Vertreter: Galander, Marcus

(56) Entgegenhaltungen:
- EP-A2- 2 332 588
- WO-A1-98/31304
- WO-A1-99/51370
- WO-A1-2010/132910
- DE-A1- 10 301 850

## Beschreibung

Die Erfindung betrifft ein Verfahren zur Herstellung eines Halbzeugs für ein Implantat, insbesondere für eine intraluminalen Endoprothese, bzw. ein Verfahren zur Herstellung eines derartigen Implantats. Medizinische Endoprothesen oder Implantate für die unterschiedlichsten Anwendungen sind in großer Vielfalt aus dem Stand der Technik bekannt. Als Implantate im Sinne der vorliegenden Erfindung sind endovaskuläre Prothesen oder sonstige Endoprothesen, beispielsweise Stents, orthopädische Implantate, z.B. Befestigungselemente für Knochen, beispielsweise Schrauben, Platten, Draht oder Nägel, chirurgisches Nahtmaterial, Darmklammern, Gefäßclips, Prothesen im Bereich des Hart- und Weichgewebes sowie Ankerelemente für Elektroden, insbesondere von Schrittmachern oder Defibrillatoren zu verstehen.

Heutzutage werden als Implantate besonders häufig Stents eingesetzt, die zur Behandlung von Stenosen (Gefäßverengungen) dienen. Sie weisen einen Körper in der Form eines rohrförmigen oder hohlzylinderförmigen Grundgitters auf, das an beiden Längsenden offen ist. Als Ausgangsform für einen solchen Körper werden in der Regel hohlzylinderförmige Halbzeuge verwendet, aus welchen dann beispielsweise mittels Laser das Grundgitter herausgeschnitten wird.

Eine derartige Endoprothese mit einem rohrförmigen Gitter wird in das zu behandelnde Gefäß eingesetzt und dient dazu, das Gefäß zu stützen. Stents haben sich insbesondere zur Behandlung von Gefäßerkrankungen etabliert. Durch den Einsatz von Stents können verengte Bereiche in den Gefäßen erweitert werden, so dass ein Lumengewinn resultiert. Durch den Einsatz von Stents oder anderen Implantaten kann zwar ein für den Therapieerfolg primär notwendiger optimaler Gefäßquerschnitt erreicht werden, allerdings initiiert die dauerhafte Anwesenheit eines derartigen Fremdkörpers eine Kaskade von mikrobiologischen Prozessen, die zu einem allmählichen Zuwachsen des Stents und im schlimmsten Fall zu einem Gefäßverschluss führen können.

Ein Ansatzpunkt zur Lösung dieses Problems besteht darin, den Stent bzw. andere Implantate aus einem biodegradierbaren Werkstoff zu fertigen.

Unter Biodegradation werden hydrolytische, enzymatische und andere stoffwechselbedingte Abbauprozesse im lebenden Organismus verstanden, die vor allem durch die mit dem biodegradierbaren Werkstoff des Implantats in Kontakt gelangenden Körperflüssigkeiten verursacht werden und zu einer allmählichen Auflösung der den biodegradierbaren Werkstoff enthaltenden Strukturen des Implantats führen. Das Implantat verliert durch diesen Prozess zu einem bestimmten Zeitpunkt seine mechanische Integrität. Synonym zu dem Begriff Biodegradation wird häufig der Begriff Biokorrosion verwendet. Der Begriff Bioresorption umfasst die anschließende Resorption der Abbauprodukte durch den lebenden Organismus.

Für den Körper biodegradierbarer Implantate geeignete Werkstoffe können beispielsweise Polymere oder Metalle enthalten. Der Körper kann dabei aus mehreren dieser Werkstoffe bestehen. Gemeinsames Merkmal dieser Werkstoffe ist ihre Biodegradierbarkeit. Beispiele für geeignete polymere Verbindungen sind Polymere aus der Gruppe Cellulose, Kollagen, Albumin, Casein, Polysaccharide (PSAC), Polylactid (PLA), Poly-L-Lactid (PLLA), Polyglykol (PGA), Poly-D,L-Lactid-co-glycolid (PDLLA-PGA), Polyhydroxybuttersäure (PHB), Polyhydroxyvaleriansäure (PHV), Polyalkylcarbonate, Polyorthoester, Polyethylenterephtalat (PET), Polymalonsäure (PML), Polyanhydride, Polyphosphazene, Polyaminosäuren und deren Copolymere sowie Hyaluronsäure. Die Polymere können je nach den gewünschten Eigenschaften in Reinform, in derivatisierter Form, in Form von Blends oder als Copolymere vorliegen. Die vorliegende Erfindung bezieht sich auf Implantate mit einem metallischen biodegradierbaren Werkstoff, insbesondere basierend auf Magnesium oder einer Magnesiumlegierung.

Es sind bereits Stents bekannt, die Beschichtungen mit verschiedenen Funktionen aufweisen. Derartige Beschichtungen dienen beispielsweise der Freisetzung von Medikamenten, der Anordnung eines Röntgenmarkers oder dem Schutz der darunter liegenden Strukturen.

Bei der Realisierung biodegradierbarer Implantate soll die Degradierbarkeit entsprechend der angestrebten Therapie bzw. der Anwendung des jeweiligen Implantats (koronar, intracranial, renal etc.) gesteuert werden. Für viele therapeutische Anwendungen ist beispielsweise ein wichtiger Zielkorridor, dass das Implantat in einem Zeitraum von vier Wochen bis sechs Monaten seine Integrität verliert. Hierbei wird unter Integrität, d. h. mechanischer Integrität, die Eigenschaft verstanden, dass das Implantat gegenüber dem undegradierten Implantat kaum mechanische Einbußen besitzt. Dies bedeutet, dass das Implantat mechanisch noch so stabil ist, dass zum Beispiel der Kollapsdruck lediglich geringfügig, d. h. höchstens auf 80% des Nominalwerts, abgefallen ist. Das Implantat kann somit bei vorhandener Integrität seiner Hauptfunktion, dem Aufhalten des Gefäßes, noch nachkommen. Alternativ kann die Integrität dadurch definiert werden, dass das Implantat mechanisch so stabil ist, dass es in seinem Belastungszustand in dem Gefäß kaum geometrischen Veränderungen unterworfen ist, beispielsweise nicht nennenswert zusammensackt, d. h. unter Belastung mindestens 80% des Dilatationsdurchmessers aufweist, oder im Fall eines Stents kaum angebrochene tragende Streben hat.

Als für den genannten Zielkorridor der Degradation besonders vielversprechend haben sich biodegradierbare Magnesium-Implantate, insbesondere Magnesium-Stents, erwiesen, die allerdings ihre mechanische Integrität bzw. Stützwirkung einerseits noch zu früh verlieren und andererseits in vitro und in vivo einen stark schwankenden Integritätsverlust aufweisen. Dies bedeutet, dass bei sich Magnesium-Stents der Kollapsdruck über die Zeit zu schnell verringert bzw. die Verringerung des Kollapsdrucks eine zu große Variabilität aufweist und damit zu unbestimmbar ist.

Im Stand der Technik sind bereits unterschiedliche Mechanismen der Degradationskontrolle von Magnesium-Implantaten beschrieben. Diese basieren beispielsweise auf anorganischen und organischen Schutzschichten oder deren Kombination, die dem humanen Korrosionsmilieu und den dort ablaufenden Korrosionsvorgängen einen Widerstand entgegensetzen. Bisher bekannte Lösungen zeichnen sich dadurch aus, dass Sperrschichtwirkungen erzielt werden, die auf einer räumlichen und möglichst defektfreien Trennung des Korrosionsmediums von dem metallischen Material, insbesondere dem metallischen Magnesium beruhen. So wird der Degradationsschutz durch verschieden zusammengesetzte Schutzschichten und durch definierte geometrische Abstände (Diffusionsbarrieren) zwischen dem Korrosionsmedium und dem Magnesiumgrundmaterial abgesichert. Weitere Lösungen auf dem Gebiet der gesteuerten Degradation rufen durch das Aufbringen von physikalischen (z.B. lokale Querschnittsveränderungen) und/oder chemischen Veränderungen der Stent-Oberfläche (z.B. lokal chemisch unterschiedlich zusammengesetzte Multilayer) Sollbrucheffekte hervor.

Ein weiteres Problem im Zusammenhang mit Beschichtungen ergibt sich auch daraus, dass Stents oder andere Implantate üblicherweise zwei Zustände annehmen, nämlich einen komprimierten Zustand mit einem kleinen Durchmesser und einen expandierten Zustand mit einem größeren Durchmesser. Im komprimierten Zustand kann das Implantat mittels eines Katheters in das zu stützende Gefäß eingeführt und an der zu behandelnden Stelle positioniert werden. Am Ort der Behandlung wird das Implantat dann beispielsweise mittels eines Ballonkatheters dilatiert. Auf Grund dieser Durchmesseränderung ist der Körper des Implantats hierbei einer mechanischen Belastung ausgesetzt. Weitere mechanische Belastungen des Implantats können während der Herstellung oder bei der Bewegung des Implantats im oder mit dem Gefäß, in das das Implantat eingesetzt ist, auftreten. Bei den genannten Beschichtungen nach dem Stand der Technik ergibt sich somit der Nachteil, dass die Beschichtung während der Verformung des Implantats reißt (z.B. Bildung von Mikrorissen) oder auch teilweise entfernt wird. Hierdurch kann eine unspezifizierte lokale Degradation verursacht werden. Außerdem sind das Einsetzen und die Geschwindigkeit der Degradation von der Größe und der Verteilung der durch die Verformung entstehenden Mikrorisse abhängig, die als Fehlstellen schlecht kontrollierbar sind. Dies führt zu einer starken Streuung in den Degradationszeiten.

Weitere Beispiele für bekannte organische oder anorganische Schutzschichten zur Erhöhung der Degradationsbeständigkeit sind galvanische Beschichtungen mittels Zink, Beschichtungen auf der Basis ionischer Flüssigkeiten, Konversionsschichten durch chemische Umwandlung der Hauptlegierungsbestandteile, Bedampfen oder Sputtern mit Aluminium, thermisches Spritzen und dergleichen, welche jedoch nur Schutzschichten sehr begrenzter Dicke (wenige µm) ergeben.

Ferner werden höher legierte Ausgangsmaterialien zur Verbesserung der Degradationsbeständigkeit verwendet. Aus der Druckschrift WO 2011/051424 A1 ist ein implantierbares medizinisches Gerät bekannt, das zumindest teilweise aus einem Material besteht, das hochreines Magnesium oder eine Magnesiumlegierung mit hochreinem Magnesium und ein oder mehrere weitere hochreine Legierungsbestandteile enthält. Hochreine Legierungsbestandteile können dabei die Elemente Indium, Scandium, Yttrium, Gallium und die Seltenen Erden darstellen. Die Herstellung von derartigen hochreinen Materialien ist sehr teuer, so dass auch die Kosten für ein daraus produziertes medizinisches Gerät hoch sind.

In der Druckschrift EP 2 332 588 wird das Kontaktieren eines Grundkörpers aus Magnesium oder einer biodegradierbaren Magnesiumlegierung mit einem korrosionsinhibierenden Element ausgewählt aus der Gruppe Al, Zn, Ca, Si, In, Mn, Sc und RE (RE steht für Seltenerd-Metalle) und eine sich daran anschließenden thermischen Behandlung beschrieben. Als Ergebnis dieses Verfahrens wird im Bereich der Kontaktfläche eine den Grundkörper bedeckende Diffusionsschicht gebildet. Die genannten Elemente diffundieren in die Oberfläche des Implantats hinein, und zwar bis zu Tiefen von etwa 20 µm. Die Eindringtiefe der jeweiligen Elemente ist durch den Diffusionskoeffizienten in der Magnesiummatrix bestimmt. Diese bekannte Lösung ist daher ebenfalls hinsichtlich der Dicke der Korrosionsschutzschicht sehr begrenzt, so dass die Anwendung dieser Technologien nur auf fertigprozessierten Implantaten sinnvoll ist. Andernfalls würde eine solche Diffusionsschicht durch nachgeschaltete Prozesse wie Reibahlen, Beizen oder Elektropolieren zumindest zum größten Teil wieder abgetragen werden.

Bei den bekannten Lösungen muss außerdem, um in den Bereich der gegenseitigen Löslichkeit der verwendeten Materialien zu gelangen, häufig eine Temperbehandlung bei Temperaturen oberhalb von 450°C (besser oberhalb von 500°C) erfolgen. In diesem Temperaturbereich findet jedoch eine Grobkornbildung in Magnesiummaterial statt, welche das mechanische Verhalten des Implantats, insbesondere deren Umformverhalten, negativ beeinflusst.

Folgende Nachteile der bekannten Lösungen bestehen daher:
1. Magnesium-Implantate ohne Oberflächenbehandlung weisen eine zu hohe Degradationsgeschwindigkeit auf.
2. Der Widerstand gegen Rissentstehung und Stegbruch beim Dilatieren von Stents aus Magnesium-Legierungen ist aufgrund der bei Körpertemperatur schwer verformbaren hexagonalen Gefügestruktur des Magnesiums gering.
3. Mögliche verwendbare Technologien zur Oberflächenbehandlung von Implantaten mit Aluminium oder Zink-Schichten, nämlich Sputtern, Bedampfen oder die Behandlung in ionischen Flüssigkeiten, sind aufwändig und die Haftung der so hergestellten Schicht auf der Magnesium-Basis ist gering.
4. Sämtliche nachträgliche Behandlungsschritte von filigranen Implantaten wie Stents sind aus produktionstechnischer Sicht nachteilig, da durch die zusätzliche Handhabung der Implantate in einem weiteren Prozessschritt häufiger mechanische Deformationen der Bauteile auftreten und dadurch eine erhöhte Ausschussrate entsteht.
5. Höher legierte Ausgangsmaterialien (z.B. Mg mit einem Legierungsanteil von Seltenen Erden von mehr als 10 Gew.%), die eine erhöhte Degradationsbeständigkeit aufweisen, haben den Nachteil, dass sie schlechtere mechanische Eigenschaften, insbesondere eine geringere Bruchdehnung, aufweisen.
6. Bei Verwendung höher legierter Ausgangsmaterialien entstehen bei der Fertigung legierungsbedingt Ausscheidungen (Inklusionen), die zu Inhomogenitäten in den mechanischen und chemischen Eigenschaften führen und dadurch eine hohe Streuung bei den mechanischen Eigenschaften bedingen.
7. Halbzeuge, die konventionell (z.B. mittels Plattierverfahren bei Blechen) hergestellt werden, zeigen bei der Verformung der daraus gefertigten Endprodukte, z.B. beim Dilatieren von Stents, häufig Delaminationen der Schicht vom Magnesium-Grundkörper.
8. Verfahren mit thermischer Nachbehandlung von Halbzeugen oder Implantaten, die mit Fremdmetallen wie Aluminium oder Zink zur Degradationskontrolle beschichtet werden, können durch Diffusion zu einer Auflegierung des Basismaterials im oberflächennahen Bereich führen. Allerdings ist dieser Bereich hinsichtlich seiner Tiefe auf wenige µm begrenzt.

Folglich besteht die Aufgabe der vorliegenden Erfindung darin, ein kostengünstiges Verfahren zur Herstellung eines Halbzeugs oder eines Implantats anzugeben, mit dem eine Degradationsverzögerung beim Implantat erreicht wird und das die obigen Nachteile nicht aufweist. Entsprechend besteht die Aufgabe der Erfindung außerdem darin, ein derartiges Halbzeug oder Implantat zu schaffen.

Die obige Aufgabenstellung wird gelöst durch Verfahren mit den Merkmalen des Anspruchs 1 und des Anspruchs 8. Das erfindungsgemäße Verfahren zur Herstellung eines Halbzeugs für ein Implantat umfasst insbesondere die folgenden Schritte:
a) Bereitstellen einer ersten Hülse aus einem ersten metallischen Werkstoff und mindestens einer zweiten Hülse aus einem zweiten metallischem Werkstoff,
b) Anordnen der ersten Hülse und der mindestens einen zweiten Hülse derart ineinander, dass diese Hülsenkombination zwischen den Hülsen eine Presspassung ausbildet,
c) Umformen der Hülsenkombination bei einer erhöhten Temperatur mittels Fließpressen.

Das erfindungsgemäße Verfahren beruht auf der Erkenntnis, dass durch Verwendung mindestens zweier ineinander gesteckter Röhrchen (oder Hohlzylinder, im Folgenden als Hülsen bezeichnet) aus unterschiedlichen Werkstoffen, die sich vorzugsweise durch eine hohe gegenseitige Mischbarkeit auszeichnen, während des erfindungsgemäß durchgeführten Umformschritts in Form einer Extrusion (Halbwarm- oder Warmfließpressen) nicht nur über mechanische Kräfte (Presspassung, z.B. eine Presspassung mit Passungstoleranzen im Bereich H7/k6, leichte Presspassung) sondern auch stofflich miteinander verbunden werden können. Durch den während des erfindungsgemäßen Verfahrens durchgeführten Umformschritt, der bei einer Temperatur des Werkzeugs und der Hülsenkombination im Bereich von 300°C bis 500°C erfolgt, bilden sich in dem Bereich, in dem die Hülsen aneinander angrenzen, abhängig von den jeweils verwendeten Materialien Werkstoffgradienten aus, da die Bestandteile des einen Materials in das jeweils andere Material hinein diffundieren. Hierdurch wird die Gefahr von Delaminationen zwischen den einzelnen Schichten reduziert, da sich zwischen den Schichten aufgrund der thermomechanischen Behandlung beim Extrusionsprozess Materialgradienten bilden und keine scharfen Grenzflächen mehr vorhanden sind. Durch die Diffusion der Materialbestandteile verzahnen die Hülsen gewissermaßen miteinander. Das erfindungsgemäße Verfahren beinhaltet daher eine thermomechanische Behandlung mit Stoffübergang.

Vorzugsweise enthält die erste Hülse Magnesium oder eine Magnesiumlegierung oder ist vollständig aus einem der beiden Materialien gefertigt. Zur Degradationskontrolle kann beispielsweise außen auf der ersten Hülse eine zweite Hülse enthaltend ein Element ausgewählt aus der Gruppe enthaltend Aluminium und Zink oder eine Legierung mit mindestens einem Element der Gruppe angeordnet sein, beispielsweise bestehend aus Al99,5 oder ZnAl4. Eine dritte Hülse aus einem der genannten Materialien zur Degradationskontrolle kann zusätzlich auch innerhalb der ersten Hülse angeordnet sein.

Werden die oben angegebenen Materialien in den außen liegenden Hüllen verwendet, so wird durch die Anreicherung der Implantatoberfläche mit diesen Materialien das elektrochemische Potential gegenüber einem Implantat aus einer Magnesiumlegierung zu höheren Werten verschoben. Dies führt zu einer verzögerten Degradationsgeschwindigkeit.

Abhängig von der Zusammensetzung der für das erfindungsgemäße Verfahren verwendeten Hülsen kann aufgrund des geringeren Anteils an Magnesium und deren Korrosionsprodukten im Zellkontakt eine geringere pH-Wert Erhöhung eingestellt werden, so dass der Abbaumechanismus gegenüber einer ggf. auf der Schicht des Halbzeugs, die durch die am weitesten außen angeordneten Hülse gebildet wurde, liegenden Polymerbeschichtung positiv beeinflusst werden kann.

Bei dem erfindungsgemäßen Verfahren ist ferner von großem Vorteil, dass sowohl die Wanddicke der für die Hülsenkombination verwendeten Röhrchen als auch deren Länge durch die Verfahrensparameter beim Extrusionsprozess und dessen Nachbehandlung in sehr weiten Grenzen variiert werden können.

Die aus dem mit dem erfindungsgemäßen Verfahren hergestellten Halbzeug gefertigten Implantate zeichnen sich durch verbesserte mechanische Eigenschaften sowohl bei der Dilatation (verringerte Bruchanfälligkeit) und als auch bei der zyklischen Belastung während des klinischen Einsatzes durch verminderte Anfälligkeit gegenüber oberflächenfehlerinduzierten Rissen sowie durch wesentlich verbesserte Korrosionseigenschaften aus.

Das erfindungsgemäße Verfahren erlaubt es nämlich, Korrosionsschutzschichten mit einer verglichen mit den oben genannten herkömmlichen Beschichtungsverfahren deutlich größeren Schichtdicke herzustellen. Dies wirkt sich auch auf die Standzeiten aus, so dass z.B. ein Stent, der auf seiner äußeren Oberfläche aus einer Aluminiumlegierung und in seiner inneren Oberfläche aus einer Magnesiumlegierung besteht, die doppelte Standzeit im Vergleich zu einem identischen, aus einer Magnesiumlegierung gefertigten Implantat besitzt.

Als Prüfmedium zur Untersuchung des Korrosionsverhaltens dient beispielsweise künstliches Plasma, das nach EN ISO 10993-15:2000 für Biokorrosionsuntersuchungen vorgeschrieben ist (Zusammensetzung NaCl 6,8 g/l, CaCl₂ 0,2 g/l, KCl 0,4 g/l, MgSO₄ 0,1 g/l, NaHCO₃ 2,2 g/l, Na₂HPO₄ 0,126 g/l, NaH₂PO₄ 0,026 g/l). Proben des zu untersuchenden Materials werden dazu in einen geschlossenen Probenbehälter mit einer definierten Menge des Prüfmediums bei 37°C gelagert. In zeitlichen Abständen - abgestimmt auf das erwartete Verhalten bei der Biodegradation - von wenigen Stunden bis zu mehreren Monaten werden die Proben entnommen und in bekannter Weise auf Degradationsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Die Konzentration des die Korrosion behindernden Materials kann zudem vergleichsweise hoch gewählt werden, so dass beispielsweise an einer aluminiumreichen äußeren Oberfläche eines Implantats erweiterte Möglichkeiten hinsichtlich einer Biofunktionalisierung, z.B. durch Anreicherung mit Phosphatverbindungen mittels plasmachemischer Oxidation, bestehen.

Die größere mechanische Robustheit aufgrund der Verwendung eines Halbzeugs mit verbesserten mechanischen Eigenschaften verringert auch die Ausschussrate bei der Fertigstellung des Implantats.

Insgesamt wird durch die erfindungsgemäße Lösung bereits auf der Stufe des Halbzeugs, das noch keine filigranen Strukturen aufweist, eine höhere mechanische und chemische Widerstandsfähigkeit erzielt, so dass die Schadenstoleranz des Gesamtsystems erhöht wird. So kann z.B. die Kerbwirkung einer sich im Magnesium-Bulk-Material angeordneten intermetallischen Inklusion minimiert werden. Derartige Inklusionen beinhalten, wenn sich diese in mechanisch hochbelasten Bereichen des Implantats befinden - eine hohe Wahrscheinlichkeit für frühe Stegbrüche an den entsprechenden Stellen. Bei Anwendung des erfindungsgemäßen Verfahrens können die oberflächennahen Randbereiche des Implantats so ausgebildet werden, dass sie eine geringere Kerbempfindlichkeit aufweisen.

In einem bevorzugten Ausführungsbeispiel wird die innen liegende erste Hülse, die auch als Basis bezeichnet wird, aus Magnesium oder einer Magnesiumlegierung gebildet. Die zweite, außen liegende Hülse enthält Aluminium oder eine Aluminiumlegierung. In diesem Fall werden gut verformbare Randschichten, insbesondere in Form von kubisch flächenzentriertem Aluminium mit mehreren Gleitebenen und ein schwerer umzuformendes Bauteilinneres aus Magnesium bzw. einer Magnesium-Legierung mit einander kombiniert. Dieses Ausführungsbeispiel zeichnet sich dadurch aus, dass ein Zuwachs an mechanischer Stabilität erreicht wird, insbesondere werden die äußeren Schichten mit Aluminium zäher und damit bruchresistenter.

In einem weiteren bevorzugten Ausführungsbeispiel wird zusätzlich eine dritte Hülse innerhalb der ersten Hülse angeordnet. Dies bedeutet, dass auch die innere Oberfläche des Halbzeugs bzw. des daraus hergestellten Implantats eine andere Zusammensetzung als die Basis aufweist.

In einem bevorzugten Ausführungsbeispiel kann die innen gelegene, dritte Hülse Zink oder eine Zink-Legierung, besonders bevorzugt ZnAl4, enthalten. Durch den Einsatz von Zink wird die Biokomptabilität verbessert. Zink hat insbesondere für orthopädische Implantate Vorteile, da ein solches Implantat aus einer Kombination aus einer das Knochenwachstum beschleunigenden Zn-Oberfläche und einer biodegradierbaren Basis besteht.

In einer Weiterbildung der Erfindung werden die erste Hülse, die mindestens eine zweite Hülse und/oder die dritte Hülse vor der Anordnung ineinander zuerst gebeizt und anschließend getrocknet. Das Beizen der Hülsen dient zur Entfernung von Kontaminationsschichten, wobei für ZnAl4 beispielsweise 5%-ige wässrige HF-Lösung, für Al99,5 beispielsweise 15%-ige NaOH-Lösung und für die Magnesiumlegierung WE43 beispielsweise 5%-ige ethanolische H₃PO₄-Lösung verwendet werden kann. Das Beizen der Hülsen wird z.B. über einen Zeitraum von etwa 1 Minute, vorzugsweise bei Raumtemperatur, durchgeführt. Die gebeizten Hülsen werden anschließend beispielsweise mittels Pressluft getrocknet und sofort ineinander gedrückt bzw. gepresst, vorzugsweise mittels einer Handhebelpresse.

Zum Umformen der Hülsenkombination mittels Fließpressen wird diese in einer im Wesentlichen hohlzylinderförmigen Matrize (auch Pressbüchse genannt) angeordnet, deren innerer Durchmesser sich am Ausgang der Matrize verringert. Der Innendurchmesser der Matrize an ihrem Ausgang entspricht dem Außendurchmesser des in der Matrize umgeformten Halbzeugs. In dem inneren Hohlraum der Hülsenkombination wird ein Stempel mit einem in Vorwärtsrichtung abragenden Dorn angeordnet, der auf die Hülsenkombination eine Presskraft in Vorwärtsrichtung ausübt und die Hülsenkombination damit durch den im Querschnitt verringerten Ausgang der Matrize drückt. Durch das Hindurchdrücken der Hülsenkombination durch den Ausgang der Matrize mit verringertem Querschnitt werden die Hülsen aufeinander gepresst, verlängert sowie in ihrem Außen- und Innendurchmesser verringert. Der Dorn ist derart angeordnet, dass er während des Pressens durch den Ausgang der Matrize hindurch ragt. Der Dorn bestimmt durch seinen Außendurchmesser den Innendurchmesser des durch den Ausgang der Matrize austretenden Halbzeugs.

Da das Umformen bei einer, wie oben bereits erläutert wurde, erhöhten Temperatur im Bereich von 300°C bis 500°C erfolgt, finden an den Grenzflächen der ineinander gesteckten Hülsen Diffusionen der jeweilige Materialkomponenten statt, so dass die aus den Hülsen entstandenen Schichten des Halbzeugs im Bereich der ursprünglichen Grenzfläche(n) Gradienten in das Innere der jeweiligen Hülse ausbilden. Vorzugsweise wird die Hülsenkombination vor Beginn des Fließpressens in dem Werkzeug über mehrere Minuten bei der erhöhten Temperatur gehalten, so dass die Hülsenkombination die Temperatur des Werkzeugs annimmt.

Durch das Umformen wird der Außendurchmesser des umgeformten Halbzeugs derart verringert, dass er höchstens 60%, vorzugsweise höchstens 55% des Außendurchmessers der Hülsenkombination vor dem Umformschritt beträgt.

Auch die gesamte Wanddicke des umgeformten Halbzeugs wird gegenüber der Wanddicke der Hülsenkombination verringert. Vorzugsweise beträgt die gesamte Wanddicke des umgeformten Halbzeugs höchstens 25%, vorzugsweise höchstens 20% der gesamten Wanddicke der Hülsenkombination vor dem Umformschritt.

Die oben dargestellte Verformung des Halbzeugs ist vergleichsweise intensiv, um die vorhandenen Grenzflächen der unterschiedlichen Metalle mechanisch und thermisch so zu zerstören, dass die Legierungsbildung durch Diffusion über die dann nicht mehr vorhandenen Grenzflächen ermöglicht wird. Bei geringeren Umformgraden vermischen sich die Metalle nicht, da die Grenzflächen dann immer noch als Diffusionsbarriere wirken können. Die erhöhte Temperatur und die vorhandene Prozesszeit reichen in diesem Fall nicht aus, um die Legierungsbildung voranschreiten zu lassen.

Die obige Aufgabenstellung wird insbesondere auch durch Verfahren zur Herstellung eines Implantats gelöst, bei dem ein Halbzeug nach dem oben beschriebenen Verfahren hergestellt und danach mittels Laser eine gewünschte Struktur, insbesondere ein Gitter, aus dem Halbzeug heraus geschnitten wird. Dieses Vorgehen ist einfach und erprobt, um aus einem Halbzeug zu dem fertigen Implantat zu gelangen.

Als weitere Bearbeitungsschritte bis zur Fertigstellung des Implantats können sich das Entgraten, Beizen, Korundstrahlen und/oder Elektropolieren nach dem Laserschneiden anschließen. Diese Schritte können zu einer Reduzierung der Schichtdicke der äußeren Schicht(en) des Halbzeugs bzw. des Implantats führen. Das Elektropolieren führt zu kontaminationsarmen Deckschichten auf Grund großer Materialabtragseffekte (Tiefenwirkung).

Ein weiterer Vorteil des mit dem erfindungsgemäßen Verfahren hergestellten Implantats besteht darin, dass dieses eine verbesserte Biokompatibilität aufweist, da die chemische Oberflächenzusammensetzung auf der luminalen und der abluminalen Seite des Implantats hinsichtlich des jeweiligen Gewebes bzw. der Körperflüssigkeit eingestellt werden kann. In einer Weiterbildung der Erfindung wird nach dem Umformen und vor dem Laserschneiden ein Temperschritt im Temperaturbereich zwischen 300°C und 500°C über mindestens 1 Minute durchgeführt und das Halbzeug anschließend vorzugsweise an Luft und vorzugsweise auf Raumtemperatur abgekühlt. Dieser Temperschritt dient dazu, in dem Gefüge nach dem Umformen enthaltene innere mechanische Spannungen abzubauen. Falls dieser Spannungsarmglühprozess nicht durchgeführt werden würde, käme es zu unerwünschten geometrischen Verformungen des Halbzeuges, die sich negativ auf die folgenden Verarbeitungsschritte auswirken würden (z.B. Taumeln in der Laserspannvorrichtung und damit verbundene erhöhte Schneidungenauigkeit beim Laserschneiden). Während des Spannungsarmglühprozesses setzt sich die oben im Rahmen des Umformschritts beschriebene Diffusion der Bestandteile der Hülsen fort.

Zusätzlich kann auf der Oberfläche des Implantats eine mehrere µm dicke Polymerbeschichtung angeordnet werden, welche als zusätzlicher Degradationsschutz und/oder zur Erhöhung der Gleitfähigkeit oder als Wirkstoffträger dient. Eine solche Polymerbeschichtung kann beispielsweise Parylene, z.B. Parylene N, und/oder PLA (Polylactid), besonders bevorzugt PLLA (Poly-L-Lactid), z.B. PLLA L210, enthalten.

Das erfindungsgemäße Verfahren kann insbesondere auch für die Herstellung von Halbzeugen in der Form von Draht und Rundstangen verwendet werden, aus denen biodegradierbare orthopädische Implantate, z.B. Knochenschrauben, -nägel, Cerclagedraht und Kirschnerdrähte mit an den Heilungsprozess adaptierten Standzeiten und besserer Umformbarkeit gefertigt werden können.

Die erfindungsgemäßen Verfahren werden nachfolgend anhand von Beispielen und Figuren erläutert.

Es zeigen schematisch:
- Fig. 1: einen Querschnitt durch eine Hülsenkombination vor dem Umformschritt und
- Fig. 2: einen Querschnitt durch das Halbzeug nach dem Umformschritt,
- Fig. 3: einen Querschnitt durch das Implantat nach dem Umformschritt und weiteren Formgebungsprozessschritten,
- Fig. 4: einen Schnitt durch das für das erfindungsgemäße Verfahren angewendete Werkzeug während des Umformens einer Hülsenkombination,
- Fig. 5: das binäre Zustandsdiagramm des Systems Magnesium-Zink und
- Fig. 6: das binäre Zustandsdiagramm des Systems Magnesium-Aluminium.

Zunächst soll darauf hingewiesen werden, dass die in der nachfolgenden Beschreibung enthaltenen Angaben zur Konzentration eines Elements, sofern an der betreffenden Stelle nichts Anderes vermerkt ist, jeweils in Masse% angegeben sind.

Eine röhrenförmige oder hohlzylinderförmige Hülse 11 aus einer Magnesiumlegierung, beispielsweise mit der Zusammensetzung WE43 (4% Y, 2% Nd, 0,5% Gd, 0,5% Dy, 0,5% Zr, Rest Mg) wird zur Herstellung eines hohlzylinderförmigen Implantat-Halbzeugs, beispielsweise für einen Stent, zunächst in der erforderlichen Länge, beispielsweise bei einer Länge von 8 mm, trocken (d.h. ohne Schmiermittel) abgedreht. Die Hülse 11 hat beispielsweise eine Wanddicke von 500 µm sowie einen Außendurchmesser von 3,4 mm. Analog werden eine hohlzylinderförmige Hülse 12 aus Al99,5 der Zusammensetzung 0,25% Si, 0,4% Fe, 0,05% Cu, 0,05% Mn, 0,05% Mg, 0,07% Zn, 0,05% Ti, Rest Aluminium (die Zusammensetzung entspricht der Zusammensetzung nach EN 573-3 und gibt Maximalgehalte (außer Al) in Masse% an) mit einer Wanddicke von 263 µm und einem Außendurchmesser Da1 von 3,926 mm und eine hohlzylinderförmige Hülse 13 aus ZnAl4 mit einer Länge von 8 mm, einer Wanddicke von 300 µm, einem Außendurchmesser von 2,4 mm und einem Innendurchmesser Di1 von 1,8 mm bereitgestellt.

Die Hülse 11 umfasst ein Volumen von 36,411 mm³, die Hülse 12 ein Volumen von 24,193 mm³ und die Hülse 13 ein Volumen von 16,064 mm³.

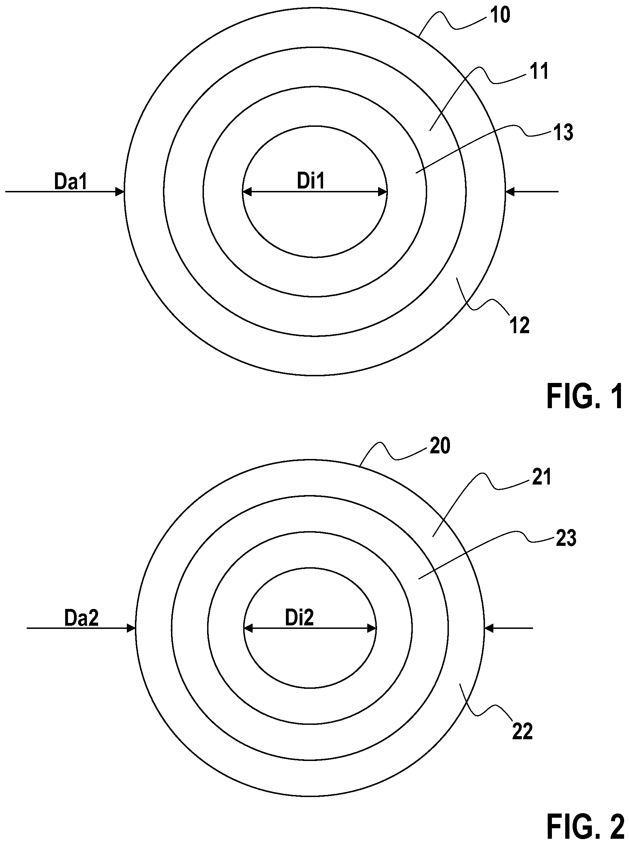

Anschließend werden die Hülsen gebeizt, und zwar Hülse 11 in 5%-iger ethanolischer H₃PO₄-Lösung, Hülse 12 in 15%-iger wässriger NaOH-Lösung und Hülse 13 in 5%-iger wässriger HF-Lösung jeweils über einen Zeitraum von 1 Minute bei Raumtemperatur. Die Hülsen 11, 12, 13 werden anschließend mittels Pressluft getrocknet und danach sofort mittels einer Handhebelpresse ineinander gedrückt, so dass die Hülsen in der in Fig. 1 gezeigten Weise als Hülsenkombination 10 ineinander angeordnet sind. Hierbei wird zunächst Hülse 13 in Hülse 11 eingepresst und anschließend die Kombination aus Hülse 11 und Hülse 13 in die außenliegende Hülse 12. Die Abmessungen der Hülsen sind derart gestaltet, dass zwischen Hülse 12 und Hülse 11 sowie zwischen Hülse 11 und Hülse 13 nach der Anordnung ineinander jeweils eine Presspassung besteht.

Bei diesem und den nachfolgend geschilderten Ausführungsbeispielen liegen die Passungstoleranzen der einzelnen Hülsensegmente im Bereich H7/k6 (leichte Presspassung). Das bedeutet, dass nur geringe Kräfte für das Ineinanderfügen der Hülsensegmente benötigt werden (bevorzugtes Übermaß beträgt ca. 6 µm, d.h. der Außendurchmesser der jeweils innen liegenden Hülse kann bis zu 6 µm größer sein als der Innendurchmesser der jeweils außen liegenden Hülse. Dies bewirkt ein Ineinanderquetschen der Rauheitsspitzen beider Hülsensegmente während des Fügeprozesses. Größere Übermaße sind nicht einzustellen, da sonst die Presskräfte zu groß werden und die Hülsen ineinander stecken bleiben, bevor sie ihre finale Position erreicht haben.

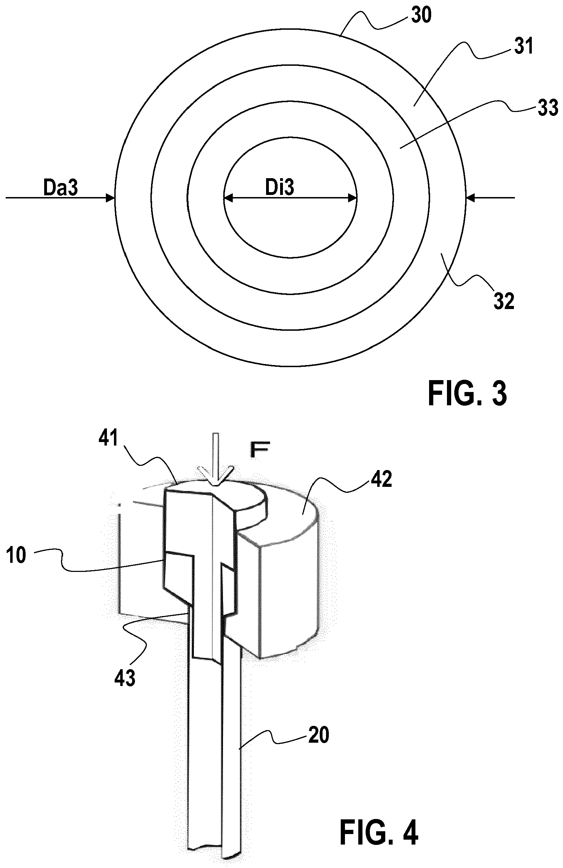

Die so hergestellte Hülsenkombination 10 wird anschließend in das in Fig. 4 gezeigte Werkzeug eingelegt, wobei das Werkzeug zuvor bereits auf eine Temperatur zwischen 300°C und 500°C aufgeheizt wurde. Hierbei wird die Hülsenkombination 10 auf den Stempel 41 aufgesteckt und in der Matrize 42 platziert. Dann wird die Hülsenkombination zunächst im Werkzeug 41, 42 über 4 Minuten bei Temperaturen zwischen 300°C und 500°C gehalten. Durch diese Maßnahme wird auch die Hülsenkombination 10 auf eine Umformtemperatur zwischen 300°C und 500°C gebracht.

Anschließend wird die Hülsenkombination wie oben beschrieben bei einer Temperatur zwischen 300°C und 500°C und einer Pressgeschwindigkeit zwischen 1 mm/min bis 100 mm/min und einer maximalen Presskraft F zwischen 10 kN und 20 kN mittels des Stempels 41 durch den Ausgang 43 der Matrize 42 hindurch gedrückt. Die Werkzeugwerkstoffe umfassen Hartmetall bzw. gehärtete Warmarbeitsstähle.

Durch das Umformen werden wie oben beschrieben die Abmessungen der Hülsenkombination verändert und es finden an den Grenzflächen der Hülsen 11 und 12 bzw. 11 und 13 die oben erläuterten Diffusionsprozesse statt, bei denen die Bestandteile der einen Hülse in die jeweils benachbarte Hülse hinein diffundieren. Durch den Umformprozess werden die oben angegebenen Volumina der Hülsen 11, 12 und 13 (nun als Schichten 21, 22, 23 bezeichnet) nicht verändert.

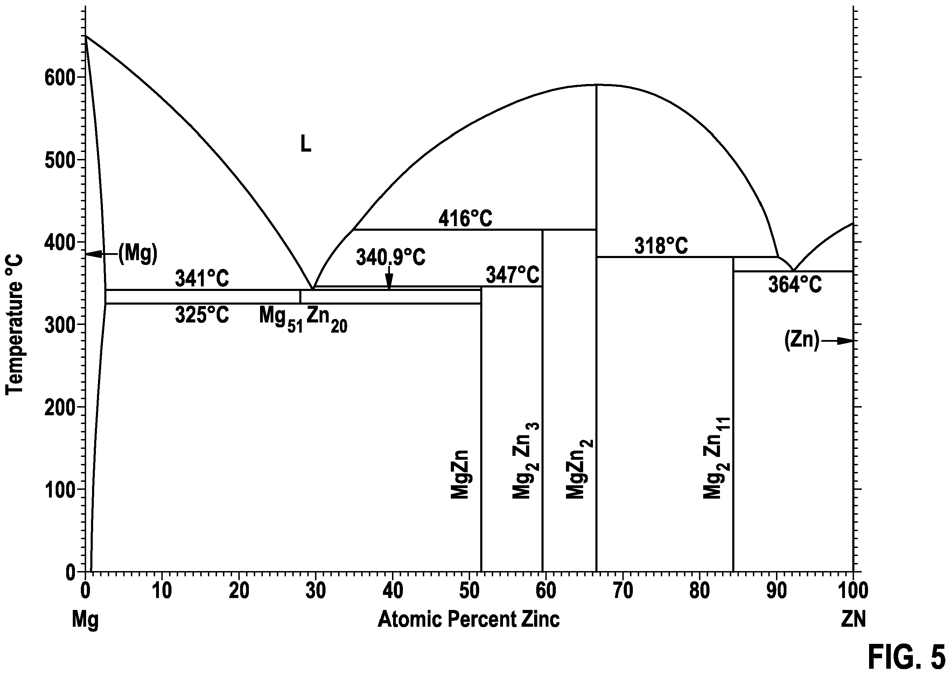

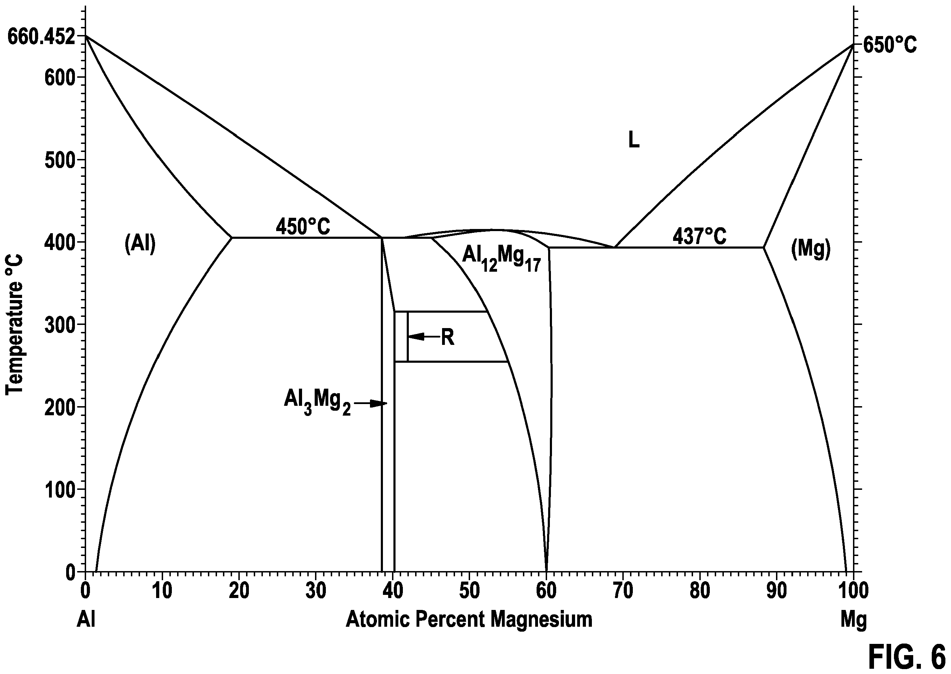

Die in den Fig. 5 und 6 dargestellten binären Zustandsdiagramme beinhalten auch die Phasengleichgewichte im Prozesstemperaturbereich zwischen 300°C und 500°C, welche das Löslichkeitsverhalten zwischen den unterschiedlichen Hülsenmaterialien veranschaulichen. Das in Fig. 5 gezeigte Zustandsdiagramm, bei dem auf der x-Achse Atom% Zink aufgetragen sind, ist vor allem für die Grenzfläche zwischen der Hülse 11 und Hülse 13 und das in Fig. 6 dargestellte Zustandsdiagramm mit der Konzentration von Mg in Atom% auf der x-Achse für die Grenzfläche zwischen der Hülse 11 und der Hülse 12 einschlägig und beschreibt das Löslichkeitsverhalten im angegebenen Temperaturbereich für die genannten Elemente.

Nach dem Fließpressschritt hat das Halbzeug 20 eine Länge von 70,8 mm. Die aus der zweiten Hülse 12 entstandene zweite Schicht 22 weist einen Außendurchmesser Da2 von 2 mm und eine Dicke von 56 µm, die aus der ersten Hülse 11 entstandene erste Schicht 21 einen Außendurchmesser von 1,888 mm und eine Dicke von 64 µm und die innen liegende, aus der dritten Hülse 13 entstandene dritte Schicht 23 einen Außendurchmesser von 1,76 mm, einen Innendurchmesser Di2 von 1,618 mm und eine Dicke von 71 µm auf. Die gesamte Dicke der Wand des Halbzeugs 20 beträgt somit 191 µm.

Anschließend wird das aus der Hülsenkombination 10 entstandene extrudierte Halbzeug 20 aus dem Werkzeug entnommen. Nun wird das Halbzeug 20 einem Spannungsarmglühschritt zwischen 300°C und 500°C über 1 Minute bis 60 Minuten unter Inertgas, beispielsweise Argon, unterzogen. Danach wird das Halbzeug auf Raumtemperatur abgekühlt.

Die weiteren Prozessschritte gestalten sich analog zu dem bekannten und vielfach beschriebenen Endabschnitt des Herstellungsverfahrens von Implantaten und umfassen finale Formgebungsprozesse wie Laserstrahlschneiden, Korundstrahlen, Entgraten, Beizen und Elektropolieren. Die Beiz- und Elektropolierschritte können z.B. in einer phosphorsäurehaltigen Lösung bei Raumtemperatur über einen Zeitraum zwischen 0,5 Minuten und 4 Minuten (bevorzugt 2 Minuten) ablaufen. Zu erwähnen ist insbesondere, dass die Prozesse Beizen, Korundstrahlen und Elektropolieren mit Materialabtrag verbunden sind. Beispielsweise beträgt der Materialabtrag beim Beizen über 2 Minuten zwischen 10 µm und 20 µm der Wanddicke.

Somit weist das Implantat 30, z.B. der Stent, am Ende des Herstellungsverfahrens noch eine Gesamtwanddicke von 166 µm auf, wobei die Dicke der zweiten Schicht 32 bei etwa 41 µm und die Dicke der dritten Schicht 33 bei etwa 61 µm liegt. Die Dicke der dazwischen liegenden ersten Schicht 31 ist unverändert gegenüber der Dicke der ersten Schicht 21 bei 64 µm des Halbzeugs 20, da die finalen Formgebungsprozesse bevorzugt an der Außenseite des Halbzeugs angreifen, wobei der Abtrag an der (äußeren) Mantelfläche des Halbzeugs 20 verfahrensbedingt mit etwa 15 µm etwas größer ist als der Materialabtrag an der Innenfläche des Halbzeugs 20 (etwa 10 µm). Somit beträgt der Außendurchmesser des Implantats 30 Da3 etwa 1,97 mm und der Innendurchmesser Di3 etwa 1,638 mm.

Weitere Möglichkeiten der Anordnung von Hülsen zu einer Hülsenkombination, die analog zu oben geschildertem Verfahren zu einem Halbzeug umgeformt und zu einem Implantat weiterverarbeitet werden können, sind nachfolgend aufgeführt. Hierbei entspricht die jeweils in der ersten Spalte angegebene Position der Hülse der in Fig. 1 gezeigten Position.

### a) abluminale Variante 1

| Position der Hülse | Werkstoff | Außendurchmesser [mm] | Innendurchmesser [mm] | Resultierende Wanddicke [µm] |
|---|---|---|---|---|
| 12 | Al99,5 | 3,925 | 3 | 462 |
| 11 | Mg WE43 | 3 | 1,8 | 600 |

Diese Variante beinhaltet ein System, bei dem das resultierende Halbzeug und das Implantat zweischichtig aufgebaut sind, wobei die äußere Schicht Al-reich ist. Ein aus dieser Variante gefertigter Stent zeichnet sich gegenüber einem Referenzstent aus massivem WE 43 durch eine wesentlich geringere Bruchanfälligkeit aus. Hierdurch kann bei einem gegebenem Stentdesign und einem Ausgangsdurchmesser von 2 mm ein maximaler Dilatationsdurchmesser von 5 mm ohne Stegbruch erzielt werden. Der Referenzstent würde im Vergleich dazu bereits bei 4,5 mm erste Stegbrüche zeigen.

### b) abluminale Variante 2

| Position der Hülse | Werkstoff | Außendurchmesser [mm] | Innendurchmesser [mm] | Resultierende Wanddicke [µm] |
|---|---|---|---|---|
| 12 | ZnAl4 | 3,925 | 3 | 462 |
| 11 | Mg WE43 | 3 | 1,8 | 600 |

Aus diesem System wird ein zweischichtiges Halbzeug hergestellt, wobei die außen liegende Schicht Zn-reich ist und ca. 4 Masse-% Al aufweist. Ein aus dieser Variante gefertigter Stent zeichnet sich gegenüber einem Referenzstent aus massivem WE 43 durch eine um 2 Monate, d.h. von ursprünglich 6 auf nunmehr 8 Monate, verlängerte Degradationszeit aus.

### c) luminale und abluminale Variante

| Position der Hülse | Werkstoff | Außendurchmesser [mm] | Innendurchmesser [mm] | Resultierende Wanddicke [µm] |
|---|---|---|---|---|
| 12 | Al99,5 | 3,925 | 3,4 | 262 |
| 11 | Mg WE43 | 3,4 | 2,4 | 500 |
| 13 | ZnAl4 | 2,4 | 1,8 | 300 |

Diese Kombination aus den Varianten a) und b) vereint die Vorteile einer geringeren Bruchanfälligkeit und einer verlängerten Degradationszeit. Es wird ein um 0,3 mm größerer Dilatationsdurchmesser und eine um 2 Monate verlängerte Degradationszeit gegenüber dem Referenzstent aus massivem WE43 erzielt. Diese Variante wurde oben anhand der Fig. 1 bis 3 dargestellt.

### d) luminale Variante 1

| Position der Hülse | Werkstoff | Außendurchmesser [mm] | Innendurchmesser [mm] | Resultierende Wanddicke [µm] |
|---|---|---|---|---|
| 11 | Mg WE43 | 3,925 | 2,4 | 762 |
| 12 | Al99,5 | 2,4 | 1,8 | 300 |

Aus diesem System kann ein zweischichtiges Halbzeug bzw. Implantat hergestellt werden, wobei die innen liegende Schicht Al-reich ist. Ein so gefertigter Stent zeichnet sich gegenüber einem Referenzstent aus massivem WE43 ebenfalls durch eine um zwei Monate verlängerte Degradationszeit aus.

### e) luminale Variante 2 (bzw. Variante für orthopädische Anwendungen)

| Position der Hülse | Werkstoff | Außendurchmesser [mm] | Innendurchmesser [mm] | Resultierende Wanddicke [µm] |
|---|---|---|---|---|
| 12 | AZ91 | 3,925 | 2,4 | 762 |
| 11 | ZnAl4 | 2,4 | 1,8 | 300 |

Aus diesen Hülsen wird ebenfalls ein zweischichtiges Halbzeug bzw. Implantat hergestellt, wobei beide Hülsen sowohl Zn als auch Al enthalten. Das für die Hülse 12 verwendete AZ91 ist eine Magnesiumbasislegierung mit 9% Al und 1 % Zn. Die innen liegende Schicht ist Zn-reich mit 4% Al.

Aus diesem Halbzeug können insbesondere kleinformatige kanülierte resorbierbare Knochenschrauben (Kleinfragmentschrauben mit einem Außendurchmesser von 2 mm) für die Versorgung distaler Radiusfrakturen hergestellt werden. Diese haben den Vorteil, dass sie durch das Knochenumfeld innerhalb von 2 Jahren vollständig absorbiert werden. Hierdurch erübrigt sich ein weiterer Eingriff zur Entfernung des Implantats. Eine weitere Anwendung stellen kanülierte Interferenzschrauben für die Rekonstruktion von Kreuzbandrupturen dar. Die genannten Schrauben zeichnen sich im Vergleich zu den klinisch derzeit verwendeten Schrauben aus degradierbaren Polymeren durch eine höhere Festigkeit aus. Hierdurch wird es möglich, mit höheren Anzugsdrehmomenten zu arbeiten, so dass der Prozess der Knochenfragmentfixation erleichtert wird.

### Bezugszeichenliste

- 10: Hülsenkombination
- 11, 12, 13: Hülse
- 20: Halbzeug
- 21, 22, 23: Schicht des Halbzeugs 20
- 30: Implantat
- 31, 32, 33: Schicht des Implantats
- 41: Stempel
- 42: Matrize
- 43: Ausgang der Matrize
- Da1: Außendurchmesser der Hülsenkombination 10
- Di1: Innendurchmesser der Hülsenkombination 10
- Da2: Außendurchmesser des Halbzeugs 20
- Di2: Innendurchmesser des Halbzeugs 20
- Da3: Außendurchmesser des Implantats 30
- Di3: Innendurchmesser des Implantats 30
- F: Presskraft

## Patentansprüche

1. Verfahren zur Herstellung eines Halbzeugs (20) für ein Implantat, insbesondere für eine intraluminale Endoprothese, umfassend die folgenden Schritte:
a) Bereitstellen einer ersten Hülse (11) aus einem ersten metallischen Werkstoff und mindestens einer zweiten Hülse (12, 13) aus einem zweiten metallischen Werkstoff,
b) Anordnen der ersten Hülse (11) und der mindestens einen zweiten Hülse (12, 13) derart ineinander, dass diese Hülsenkombination (10) zwischen den Hülsen mindestens eine Presspassung ausbildet,
c) Umformen der Hülsenkombination (10) bei einer erhöhten Temperatur mittels Fließpressen, wobei
das Umformen bei einer Temperatur des Werkzeugs und der Hülsenkombination (10) im Bereich von 300°C bis 500°C erfolgt.

2. Verfahren nach Anspruch 1, **dadurch gekennzeichnet, dass** die erste Hülse (11) Magnesium oder eine Magnesiumlegierung enthält.

3. Verfahren nach einem der vorhergehenden Ansprüche, **dadurch gekennzeichnet, dass** die erste Hülse (11) in der zweiten Hülse (12) angeordnet wird.

4. Verfahren nach einem der vorhergehenden Ansprüche, **dadurch gekennzeichnet, dass** die zweite oder eine dritte Hülse (13) in der ersten Hülse (11) angeordnet wird.

5. Verfahren nach einem der vorhergehenden Ansprüche, **dadurch gekennzeichnet, dass** die erste Hülse (11) und die mindestens eine zweite Hülse (12, 13) vor der Anordnung ineinander zuerst gebeizt und anschließend getrocknet werden.

6. Verfahren nach einem der vorhergehenden Ansprüche, **dadurch gekennzeichnet, dass** der Außendurchmesser (Da2) des umgeformten Halbzeugs (20) höchstens 60%, vorzugsweise höchstens 55% des Außendurchmessers (Da1) der Hülsenkombination (10) vor dem Fließpressen beträgt.

7. Verfahren nach einem der vorhergehenden Ansprüche, **dadurch gekennzeichnet, dass** der gesamte Wanddicke des umgeformten Halbzeugs (20) höchstens 25%, vorzugsweise höchstens 20% der gesamten Wanddicke der Hülsenkombination (10) vor dem Fließpressen beträgt.

8. Verfahren zur Herstellung eines Implantats, vorzugsweise einer intraluminalen Endoprothese, **dadurch gekennzeichnet, dass** ein Halbzeug (20) nach einem in den vorhergehenden Ansprüchen angegebenen Verfahren hergestellt wird und danach mittels Laser aus dem Halbzeug (20) eine gewünschte Struktur, insbesondere ein Gitter herausgeschnitten wird.

9. Verfahren zur Herstellung eines Implantats nach Anspruch 8, **dadurch gekennzeichnet, dass** mit dem Halbzeug (20) nach dem Umformen und vor dem Laserschneiden ein Temperschritt in einem Temperaturbereich zwischen 300°C und 500°C über mindestens 1 Minute durchgeführt wird.

10. Verfahren zur Herstellung eines Implantats nach Anspruch 8, **dadurch gekennzeichnet, dass** aus dem Halbzeug (20) ein orthopädisches Implantat hergestellt wird.

## Claims

1. A method for producing a semifinished product (20) for an implant, in particular for an intraluminal endoprosthesis, comprising the following steps:
a) providing a first sleeve (11) from a first metallic material and at least one second sleeve (12, 13) from a second metallic material,
b) arranging the first sleeve (11) and the at least one second sleeve (12, 13) one inside the other in such a manner that this sleeve combination (10) forms at least a press fit between the sleeves, and
c) forming the sleeve combination (10) at an increased temperature by means of extrusion, wherein
the forming is performed at a temperature of the tool and of the sleeve combination (10) in the range of from 300°C to 500°C.

2. The method according to claim 1, **characterised in that** the first sleeve (11) contains magnesium or a magnesium alloy.

3. The method according to either one of the preceding claims, **characterised in that** the first sleeve (11) is arranged in the second sleeve (12).

4. The method according to any one of the preceding claims, **characterised in that** the second or a third sleeve (13) is arranged in the first sleeve (11).

5. The method according to any one of the preceding claims, **characterised in that** the first sleeve (11) and the at least one second sleeve (12, 13) are first etched and subsequently dried prior to being arranged one inside the other.

6. The method according to any one of the preceding claims, **characterised in that** the outer diameter (Da2) of the formed semifinished product (20) is at most 60%, preferably at most 55% of the outer diameter (Da1) of the sleeve combination (10) prior to the extrusion.

7. The method according to any one of the preceding claims, **characterised in that** the total wall thickness of the formed semifinished product (20) is at most 25%, preferably at most 20% of the total wall thickness of the sleeve combination (10) prior to the extrusion.

8. A method for producing an implant, preferably an intraluminal endoprosthesis, **characterised in that** a semifinished product (20) is produced by a method specified in the preceding claims and then a desired structure, in particular a mesh, is cut by means of laser out of the semifinished product (20).

9. The method for producing an implant according to claim 8, **characterised in that**, after the forming and prior to the laser cutting, a tempering step is carried out with the semifinished product (20) for at least 1 minute in a temperature range between 300°C and 500°C.

10. The method for producing an implant according to claim 8, **characterised in that** an orthopaedic implant is produced from the semifinished product (20).

## Revendications

1. Procédé de fabrication d'un demi-produit (20) destiné à un implant, notamment, destiné à une endoprothèse intraluminale, comprenant les étapes suivantes :
a) de mise à disposition d'un premier manchon (11) à base d'un premier matériau métallique et d'au moins un deuxième manchon (12, 13) à base d'un deuxième matériau métallique,
b) d'agencement du premier manchon (11) et de l'au moins un deuxième manchon (12, 13), l'un dans l'autre, de telle manière que cette combinaison des manchons (10) forme au moins un ajustement par serrage entre les manchons,
c) de remodelage de la combinaison des manchons (10) à température élevée au moyen d'une extrusion sous pression, où
le remodelage a lieu à une température de l'outil et de la combinaison des manchons (10) dans la plage de 300 °C à 500 °C.

2. Procédé selon la revendication 1, **caractérisé en ce que** le premier manchon (11) contient du magnésium ou un alliage de magnésium.

3. Procédé selon l'une des revendications précédentes, **caractérisé en ce que** le premier manchon (11) est disposé dans le deuxième manchon (12).

4. Procédé selon l'une des revendications précédentes, **caractérisé en ce que** le deuxième ou un troisième manchon (13) est disposée ou sont disposés dans le premier manchon (11).

5. Procédé selon l'une des revendications précédentes, **caractérisé en ce que** le premier manchon (11) et l'au moins un deuxième manchon (12, 13) avant l'agencement de l'un dans l'autre sont d'abord décapés et ensuite séchés.

6. Procédé selon l'une des revendications précédentes, **caractérisé en ce que** le diamètre extérieur (Da2) du demi-produit (20) remodelé est au maximum égal à 60 %, de préférence, au maximum égal à 55 % du diamètre extérieur (Da1) de la combinaison des manchons (10) avant l'extrusion sous pression.

7. Procédé selon l'une des revendications précédentes, **caractérisé en ce que** l'épaisseur de la paroi totale du demi-produit (20) remodelé est égal au maximum à 25 %, de préférence, au maximum à 20 % de l'épaisseur de la paroi totale de la combinaison des manchons (10) avant l'extrusion sous pression.

8. Procédé de fabrication d'un implant, de préférence, d'une endoprothèse intraluminale, **caractérisé en ce**
**qu'**un demi-produit (20) est fabriqué selon le procédé indiqué dans les revendications précédentes et qu'ensuite une structure souhaitée, notamment une grille, est découpée à partir du demi-produit (20) au moyen d'un laser.

9. Procédé de fabrication d'un implant selon la revendication 8, **caractérisé en ce qu'**avec le demi-produit (20), après le remodelage et avant la découpe par laser, on effectue une étape de recuit dans une plage de température entre 300 °C et 500 °C pendant au moins 1 minute.

10. Procédé de fabrication d'un implant selon la revendication 8, **caractérisé en ce qu'**à partir du demi-produit (20) on fabrique un implant orthopédique.
